# EUROPÄISCHE PATENTSCHRIFT

(11) **EP 2 499 231 B1**
(45) Veröffentlichungstag und Bekanntmachung des Hinweises auf die Patenterteilung: **20.09.2017**
(21) Anmeldenummer: 10787993.4
(22) Anmeldetag: 28.10.2010
(51) Int. Cl.: C12M 1/04, B01F 3/04

(54) **BEGASUNGSVORRICHTUNG FÜR BIOREAKTOREN**
GASSING DEVICE FOR BIOREACTORS
DISPOSITIF D'APPORT DE GAZ POUR BIORÉACTEURS

(30) Priorität: 12.11.2009 DE 102009052670
(43) Veröffentlichungstag der Anmeldung: 19.09.2012
(73) Patentinhaber: Sartorius Stedim Biotech GmbH, 37079 Göttingen (DE)
(72) Erfinder: PRADEL, Günter, 37077 Göttingen (DE); WEISSHAAR, Stefan, 37139 Adelebsen (DE); GRELLER, Gerhard, 37085 Göttingen (DE); NOACK, Ute, 37079 Göttingen (DE)
(74) Vertreter: Schneider, Peter Christian
(86) Internationale Anmeldenummer: PCT/EP2010/006601
(87) Internationale Veröffentlichungsnummer: WO 2011/057718

(56) Entgegenhaltungen:
- EP-A1- 1 884 561
- WO-A2-2008/088371
- US-A- 4 207 275
- US-A- 4 557 879
- US-A- 5 241 992

## Beschreibung

### Gebiet der Erfindung

Die Erfindung betrifft eine Begasungsvorrichtung für Bioreaktoren mit einem ersten Begasungselement mit Gasaustrittsöffnungen einer ersten Größe und mit mindestens einem zweiten Begasungselement mit Gasaustrittsöffnungen einer zweiten Größe, und wobei die Begasungselemente mit getrennten Begasungskanälen gebildet sind.

### Stand der Technik

Die Sauerstoffversorgung ist ein wichtiger Schlüssel bei zellulären Stoffwechselvorgängen. Obwohl Tierzellkulturen wesentlich weniger Sauerstoff verbrauchen als Bakterien und Hefekulturen, ist die effiziente Versorgung die größte Herausforderung beim Betrieb eines Zellkultur-Bioreaktors. Neben der Sauerstoffversorgung der Zellen spielt auch die Konzentration von gelöstem Kohlendioxid als Regelgröße eine Rolle.

Es gibt zwei klassische Begasungsmethoden: Die Begasung des Kopfraumes des Bioreaktors und das direkte Einblasen der Gase durch Begasungsringe. Angewendet werden dabei sowohl die aus Fermentern bekannten Begasungsringe mit Bohrungen bzw. Gasaustrittsöffnungen von z.B. 0,8 mm als auch so genannte Mikrosparger aus gesinterten Kunststoffen mit Porengrößen von z.B. 20 bis 45 µm, die ebenfalls Gasaustrittsöffnungen bilden. Beide Typen haben spezielle Vor- bzw. Nachteile. Der Begasungsring erzeugt größere Blasen, daher sind für die gleiche "Sauerstofftransferrate" größere Gasdurchsatzraten nötig. Der Ringsparger ist mit seinen relativ großen Blasen zum CO₂-Strippen bzw. Austreiben durch z.B. Luft geeignet. Der Mikrosparger mit seinen relativ kleinen Blasen ist besonders zur Sauerstoffversorgung geeignet. Nachteilig dabei ist jedoch, dass es wegen der relativ kleineren Blasen unter ungünstigen Bedingungen zur Schaumbildung kommen kann.

Die Begasungsvorrichtungen werden als Teil von automatisierten Begasungssystemen von Bioreaktoren, beispielsweise Einwegreaktoren eingesetzt, wobei die Zufuhr von Luft, Sauerstoff, Kohlendioxid und Stickstoff unabhängig voneinander regelbar ist. Sensoren für den SauerstoffPartialdruck und den pH-Wert ermöglichen die Regelung dieser wichtigen Prozessparameter.

Aus der WO 2009/122310 A2, WO 2009/115926 A2 und der WO 2009/116002 A1 ist ein Einweg-Bioreaktor mit einem Mischer und mit einer am Boden des Reaktorinnenraumes angeordneten Begasungsvorrichtung bekannt. Dabei ist es bekannt, am Boden zwei Begasungselemente anzuordnen, die als gegenüberliegende ineinander greifende Ringsegmente ausgebildet sind.

Nachteilig bei dieser Begasungsvorrichtung, die sich grundsätzlich bewährt hat, ist, dass sie zum einen relativ aufwendig am Reaktorboden zu befestigen ist und dass es zum anderen schwierig ist, eine Mehrzahl solcher Begasungselemente optimal zum Rührer bzw. Mischer anzuordnen.

Weiterhin ist aus der WO 2008/088371 A2 eine Begasungsvorrichtung für Bioreaktoren bekannt, bei der beispielsweise zwei Begasungselemente mit unterschiedlichen Gasaustrittsöffnungen am Reaktorboden befestigbar sind. Die unterschiedlichen Begasungselemente können unabhängig voneinander mit unterschiedlichem Gas versorgt werden

Nachteilig bei der bekannten Vorrichtung ist, dass die Begasungselemente unterschiedliche Gehäuse aufweisen, die relativ aufwendig am oder in dem Reaktorboden befestigt sind, wobei die Gaszuflüsse zu den Begasungselementen in vertikaler Richtung von unten her über den Reaktorboden zugeführt werden.

Weiterhin ist aus der US 4 207 275 A eine Begasungsvorrichtung zum Mischen von Flüssigkeit mit Gas in einem Flüssigkeitstank bekannt, die eine Umwälzeinrichtung zur Erzeugung einer Radialströmung der Flüssigkeit im Tank aufweist. Zwei ringförmige Begasungselemente sind dabei in einem gemeinsamen Gehäuse angeordnet. Die Begasungselemente können an ihrer Oberseite unterschiedlich große Gasaustrittsöffnungen aufweisen.

Nachteilig ist auch hier, dass die Zuführung von Gas zu den Begasungselementen jeweils über eine durch den Boden des Behälters hindurchgeführte Leitung über den Boden des Gehäuses der Begasungselemente in diese eingeführt werden soll. Weiterhin nachteilig ist, dass die Begasungselemente dabei über Klammern oder Halter am Reaktorboden relativ aufwendig in einem Abstand befestigt sind.

Aus der US 5 241 992 A ist eine Begasungsvorrichtung mit mehreren konzentrisch zueinander angeordneten Begasungselementen bekannt. Die Begasungselemente sind in einem gemeinsamen Gehäuse als ringförmige Kanäle in vertikaler Richtung übereinander und zueinander beabstandet angeordnet. Die Begasungselemente weisen radial verlaufende Zuflüsse auf, die ebenfalls in vertikaler Richtung übereinander angeordnet sind.

Nachteilig dabei ist, dass die Gasaustrittsöffnungen der einzelnen Begasungselemente jeweils über Gaskanäle zu einem gemeinsamen Austrittspunkt geführt werden. Einerseits wird bei dieser bekannten Vorrichtung durch die in radialer Richtung verlaufenden Zuflüsse eine Zuleitung über den Reaktorboden vermieden, andererseits wird aber durch die Anordnung der Begasungselemente in vertikaler Richtung übereinander ein relativ voluminöses und kompliziert aufgebaut gemeinsames Gehäuse benötigt.

### Aufgabenstellung

Aufgabe der vorliegenden Erfindung ist es daher, die bekannten Begasungsvorrichtungen für Bioreaktoren so zu verbessern, dass sie einerseits einfacher zu befestigen sind und andererseits jedes Begasungselement eine optimale Position zu einem im Reaktorinnenraum angeordneten Mischelement aufweist.

### Darstellung der Erfindung

Diese Aufgabe wird in Verbindung mit dem Oberbegriff des Anspruches 1 dadurch gelöst, dass die Begasungselemente von einem gemeinsamen Gehäuse gebildet werden, dass das Gehäuse als eine ringförmige Scheibe ausgebildet ist, in der die Begasungselemente konzentrisch zueinander angeordnet sind, dass das Gehäuse ein Unterteil aufweist, in dem die Begasungskanäle mit jeweils einem radial verlaufenden Zufluss angeordnet sind, dass die Begasungskanäle zueinander hin von einer Zwischenwandung und in vertikaler Richtung nach unten von einem Boden begrenzt sind.

Durch das gemeinsame Gehäuse und die Ausbildung als eine ringförmige Scheibe, in der die mindestens zwei Begasungselemente konzentrisch zueinander angeordnet sind, lässt sich die Begasungsvorrichtung bei geringem Platzbedarf einfach und zentral zu einem Mischer anordnen. Damit müssen weder die Begasungselemente noch die Zuflüsse durch den Reaktorboden geführt werden. Die Begasungselemente können dabei wegen des gemeinsamen Gehäuses mit den radial verlaufenden Zuflüssen relativ einfach zwischen Reaktorboden und Mischer angeordnet und optimal zum Mischer ausgerichtet werden.

Nach einer bevorzugten Ausführungsform der Erfindung ist das erste Begasungselement als ein Ringsparger mit Gasaustrittsöffnungen von größer als 0,1 mm Durchmesser ausgebildet, während das zweite Begasungselement als ein Mikrosparger mit Gasaustrittöffnungen von kleiner als 0,1 mm Durchmesser ausgebildet ist. Dabei werden die Gasaustrittsöffnungen des Mikrospargers von einem porösen Material, beispielsweise gesinterten Kunststoff, gebildet. Damit sind auf engem Raum in einem gemeinsamen Gehäuse sowohl ein Ringsparger als auch ein Mikrosparger platzsparend angeordnet.

Gemäß einer weiteren bevorzugten Ausführungsform der Erfindung ist das erste Begasungselement und das zweite Begasungselement als Ringsparger mit jeweils zwei verschieden großen Gasaustrittsöffnungen ausgebildet, wobei die Gasaustrittsöffnungen größer als 0,1 mm sind.

Gemäß einer weiteren bevorzugten Ausführungsform der Erfindung ist das erste Begasungselement und das zweite Begasungselement als Microsparger mit jeweils zwei verschieden großen Gasaustrittsöffnungen ausgebildet, wobei die Gasaustrittsöffnungen kleiner als 0,1 mm sind.

Gemäß einer weiteren bevorzugten Ausführungsform der Erfindung ist das Unterteil von einem Oberteil abgedeckt, das den Begasungskanal des Ringspargers abdeckt und die Gasaustrittsöffnungen aufweist.

Im Bereich des Mikrospargers weist das Oberteil zum Begasungskanal hin segmentförmige Öffnungen auf, wobei der Begasungskanal von einem ringförmigen Belüftungsring aus dem die Gasaustrittsöffnungen bildenden porösen Material abgedeckt bzw. abgedichtet ist. Grundsätzlich ist es aber auch möglich, den Belüftungsring direkt in das Oberteil einzusetzen.

Das Oberteil lässt sich gegenüber dem Unterteil relativ einfach durch Dichtungen, beispielsweise Schnurringdichtungen bzw. O-Ringe, abdichten.

Bei weiteren bevorzugten Ausführungsformen kann das Oberteil mit dem Unterteil entweder verschweißt oder verklebt sein.

Gemäß einer weiteren bevorzugten Ausführungsform der Erfindung weisen die Zuflüsse zur unabhängigen Versorgung der Begasungskanäle jeweils einen Schlauchanschluss mit einem Rückschlagventil auf. Die Rückschlagventile verhindern zuverlässig einen Medienrückfluss aus dem Reaktorinnenraum in die Begasungsvorrichtung.

Gemäß einer weiteren bevorzugten Ausführungsform der Erfindung weist das Gehäuse eine zentrale auf einen Rührerflansch des Bioreaktors abgestimmte Öffnung auf und ist einem Rührer vorgelagert am Boden eines Innenraums des Bioreaktors anordenbar. Durch die zentrale Öffnung der Begasungsvorrichtung ist diese optimal zu einem im Reaktorinnenraum angeordneten Rührer positionierbar.

Durch unterschiedliche Größen können die Volumenströme der Begasungselemente an das Reaktorvolumen angepasst werden. Insbesondere ist bei dem Ringsparger die Lochzahl auf das Reaktor- bzw. Nutzvolumen abstimmbar. Beispielsweise kann je 10 Liter Nutzvolumen ein Loch im Ringsparger angeordnet sein. Bei dem Mikrosparger wird dessen Fläche ebenfalls an das Reaktorvolumen angepasst, beispielsweise 2 bis 2,5 cm² je 10 Liter Nutzvolumen. Damit kann eine konstante Blasengrößenverteilung bei beliebiger Skalierbarkeit des Reaktorvolumens erreicht werden.

Neben der Anzahl der Gasaustrittsöffnungen bzw. Löcher kann auch deren Größe den gewünschten Volumenströmen angepasst werden. Entsprechend kann bei den Mikrospargern die Porosität und deren Fläche angepasst werden.

Nach einer weiteren bevorzugten Ausführungsform der Erfindung weist das Unterteil einen durchgehenden, kreisförmigen Boden auf und ist durch Schraub-, Bajonett-, Rast-, Klemm-, Kleb-oder Klippverbindung dichtend in den Boden des Bioreaktors einsetzbar.

Weitere Einzelheiten der Erfindung ergeben sich aus der nachfolgenden ausführlichen Beschreibung und den beigefügten Zeichnungen, in denen bevorzugte Ausführungsformen der Erfindung beispielhaft veranschaulicht sind.

### Kurzbeschreibung der Zeichnungen

In den Zeichnungen zeigen:

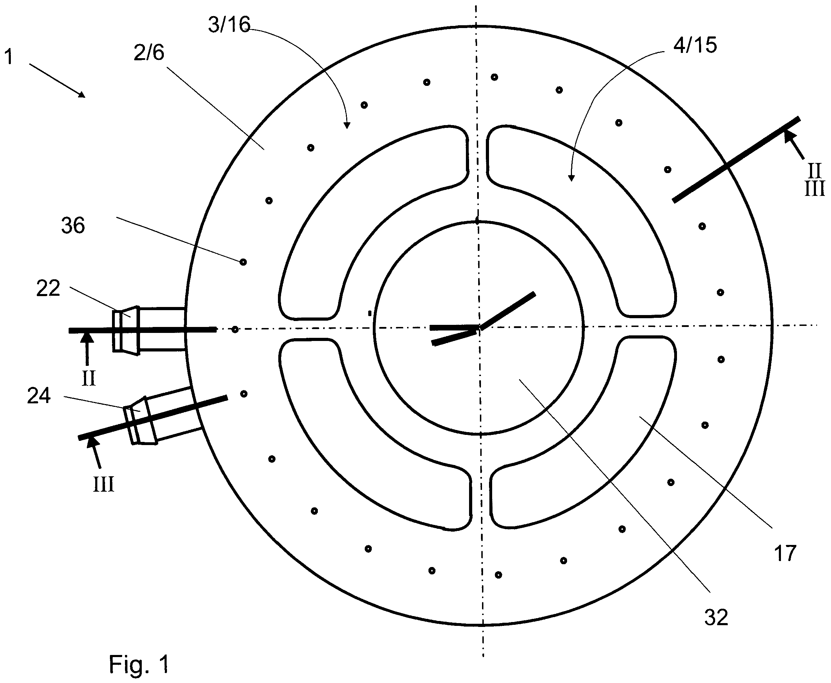

- Figur 1:: eine Draufsicht auf eine Begasungsvorrichtung mittlerer Größe in vergrößerter Darstellung,
- Figur 2:: eine Seitenansicht der Begasungsvorrichtung von Fig. 1 entlang der Linie II - II geschnitten,
- Figur 3:: eine Seitenansicht der Begasungsvorrichtung von Fig. 1 entlang der Linie III - III geschnitten,
- Figur 4:: eine Draufsicht auf eine weitere Begasungsvorrichtung mit gegenüber der Begasungsvorrichtung von Fig. 1 kleinerer Größe,
- Figur 5:: eine Draufsicht auf eine weitere Begasungsvorrichtung mit gegenüber der Begasungsvorrichtung von Fig. 1 größerer Größe und
- Figur 6:: eine Seitenansicht eines Bioreaktors mit Rührer und einer Begasungsvorrichtung.
- Figur 7:: eine Seitenansicht eines weiteren Bioreaktors mit einer Begasungsvorrichtung deren Unterteil über einen durchgehenden Boden durch eine Schraubverbindung in den Boden des Bioreaktors einsetzbar ist und.
- Figur 8:: eine Seitenansicht eines weiteren Bioreaktors mit einer Begasungsvorrichtung deren Unterteil über einen durchgehenden Boden durch eine Rastverbindung in den Boden des Bioreaktors einsetzbar ist.

### Beschreibung der Ausführungsbeispiele

Eine Begasungsvorrichtung 1 besteht im Wesentlichen aus einem Gehäuse 2 mit einem ersten Begasungselement 3 und einem zweiten Begasungselementen 4.

Das Gehäuse 2 besteht aus einem Unterteil 5 und einem auf das Unterteil 5 aufsetzbaren Oberteil 6. Das Unterteil 5 weist zwei konzentrisch zueinander angeordnete Begasungskanäle 7, 8 auf. Die Begasungskanäle 7,8 sind in vertikaler Richtung nach unten von einem Boden 9 des Unterteils 5 begrenzt. Der erste Begasungskanal 7 wird seitlich von der äußeren Wandung 10 und zum zweiten Begasungskanal 8 hin von einer Zwischenwandung 11 begrenzt. Entsprechend wird der zweite Begasungskanal 8 zum ersten Begasungskanal 7 hin von der Zwischenwandung 11 und auf seiner dem ersten Begasungskanal 7 abgewandten Seite durch eine innere Wandung 12 des Unterteils 5 begrenzt.

Der zweite Begasungskanal 8 weist auf seiner dem Boden 9 abgewandten Seite einen ringförmigen Absatz 13 auf, in den ein ringförmiger Belüftungsring 14 einsetzbar ist, der den zweiten Begasungskanal 8 in vertikaler Richtung nach oben hin abschließt. Der Belüftungsring 14 besteht aus einem gesinterten porösen Material, das die Gasaustrittsöffnungen des zweiten Begasungselementes 4 bildet, das als ein sogenannter Mikrosparger 15 ausgebildet ist. Entsprechend weist der Belüftungsring 14 Gasaustrittsöffnungen auf, deren Durchmesser kleiner als 0,1 mm ist.

Dass auf das Unterteil 5 aufsetzbare Oberteil 6 verschließt den ersten Begasungskanal 7 und bildet mit diesem das erste Begasungselement 3. Hierzu weist das Oberteil 6 im Bereich des Begasungskanals 7 Gasaustrittsöffnungen 36 auf, deren Durchmesser größer als 0,1 mm ist. Das erste Begasungselement 3 bildet dabei einen so genannten Ringsparger 16.

Das Oberteil 6 weist im Bereich des zweiten Begasungskanals 8 segmentförmige Öffnungen 17 auf, die den Belüftungsring 14 in seinen wesentlichen Bereichen mit den Gasaustrittsöffnungen 36 freigeben.

Zur Abdichtung zwischen Unterteil 5 und Oberteil 6 sind auf den Wandungen 10, 11, 12 jeweils Dichtungen 18, 19, 20 angeordnet, die als Schnurringdichtungen bzw. O-Ringe ausgebildet sind.

Der erste Begasungskanal 7 weist einen radial verlaufenden ersten Zufluss 21 auf, der in einen Schlauchanschluss 22 mündet. Entsprechend weist der zweite Begasungskanal 8 einen zweiten radial verlaufenden Zufluss 23 auf, der in einen Schlauchanschluss 24 mündet. In den Schlauchanschlüssen 22, 24 oder in den Zuflüssen 21, 23 können nicht dargestellte Rückschlagventile angeordnet sein.

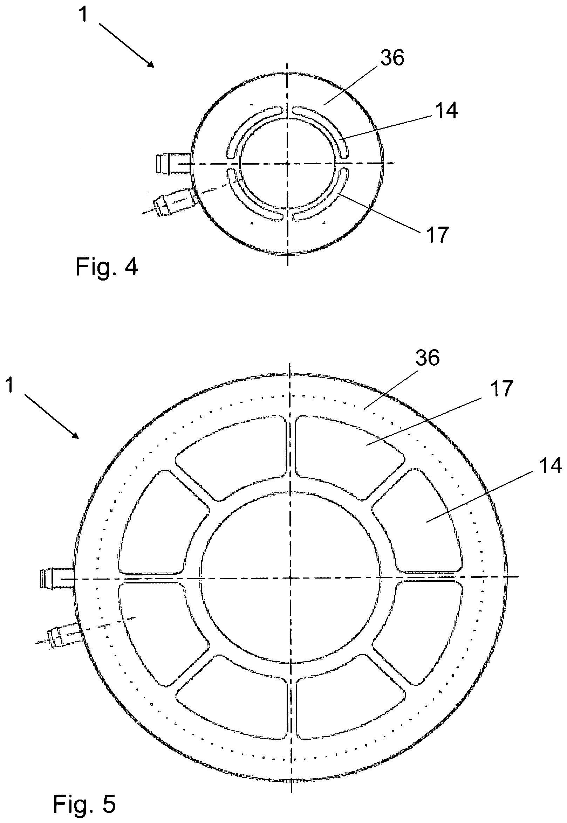

Die Figuren 4 und 5 zeigen zwei weitere Ausführungsbeispiele der Begasungsvorrichtung, die eine beliebige Skalierbarkeit der Begasungsvorrichtung für den Übergang von einem kleineren auf ein größeres Reaktornutzvolumen ermöglichen. Diese Skalierbarkeit wird zum einen durch die Variation der Anzahl der Gasaustrittsöffnungen 36 im Ringsparger 3 erreicht und zum anderen durch die Anzahl und Flächenausdehnung der segmentförmigen Öffnungen 17 des Belüftungsrings 14, in welchem sich das poröse, gasdurchlässige Material befindet. Bevorzugt weist der Belüftungsring 14 eine Gasdurchtrittsöffnung 36 pro 10 l Reaktornutzvolumen auf, während die Flächenausdehnung der segmentfömigen Öffnungen 17 mit porösem Material bevorzugt 2 bis 2,5 cm² pro 10 l Reaktornutzvolumen beträgt.

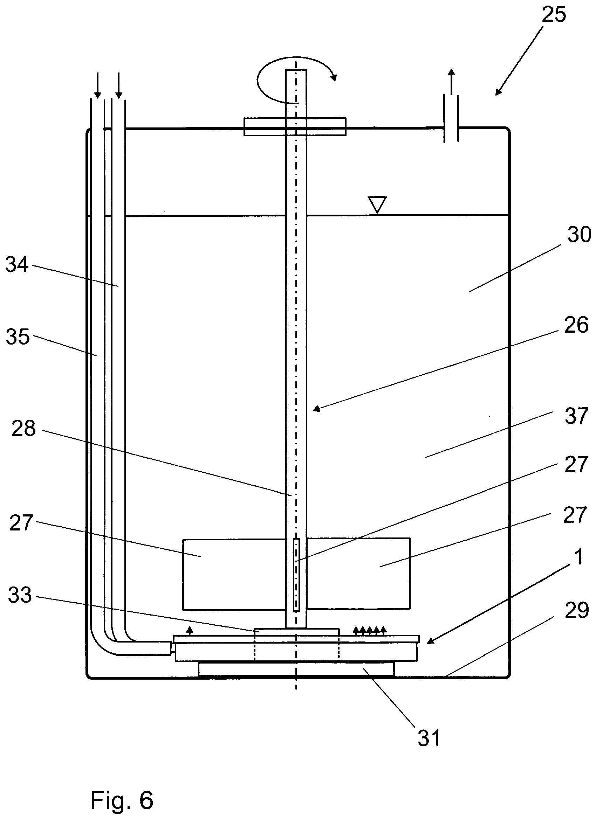

Figur 6 zeigt beispielhaft einen Biorektor 25, der beispielsweise als ein flexibler Beutel zum Einmalgebrauch ausgebildet und einen von außerhalb antreibbaren Rührer 26 mit Rührerblättern 27 aufweist. Der Bioreaktor 25 weist einen Innenraum 30 auf, an dessen Boden 29 ein Rührerflansch 31 angeordnet ist. In dem Rührerflansch ist der Rührerschaft 28 drehbar gelagert. Mit ihrer zentralen Öffnung 32 ist die Begasungsvorrichtung 1 auf einen Bund 33 des Rührerflansches 31 aufgesteckt. Über eine erste Zuflussleitung 34 wird dem ersten Zufluss 21 und über eine zweite Zuflussleitung 35 wird dem zweiten Zufluss 23 Gas zugeführt, das aus der Begasungsvorrichtung 1 austritt und in dem flüssigen Medium 37 im Innenraum 30 Blasen bildet.

Weitere vorhandene Zu- und Abflüsse sowie Steuer- und Regeleinrichtungen sind nicht dargestellt.

In einer bevorzugten Ausführungsform beträgt das Dimensionierungsverhältnis zwischen dem Durchmesser der Begasungsvorrichtung 1 und dem Durchmesser des Bioreaktors 25 bevorzugt zwischen 0,1 und 0,5, besonders bevorzugt 0,240. Das Dimensionierungsverhältnis zwischen der Begasungsvorrichtung 1 und dem Durchmesser der Rührblätter 27 beträgt zwischen 0,4 und 0,7, bevorzugt 0,622. Bei einer bevorzugten Bauform der Begasungseinrichtung ist das Ober- und Unterteil der Begasungseinrichtung aus mit Gammastrahlung sterilisierbarem Kunststoff, bevorzugt Polycarbonat, gefertigt, während das poröse Material in den segmentförmigen Öffnungen 17 aus Polyethen besteht.

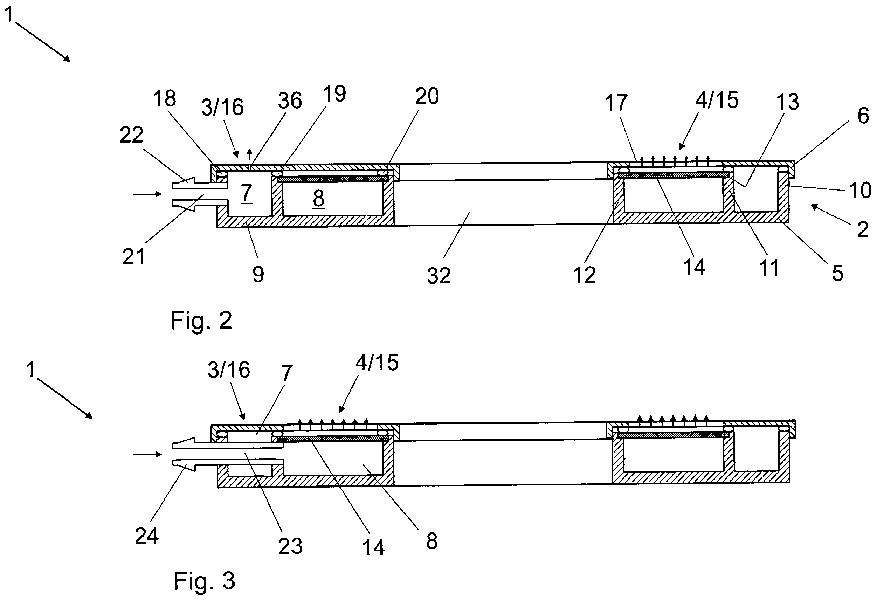

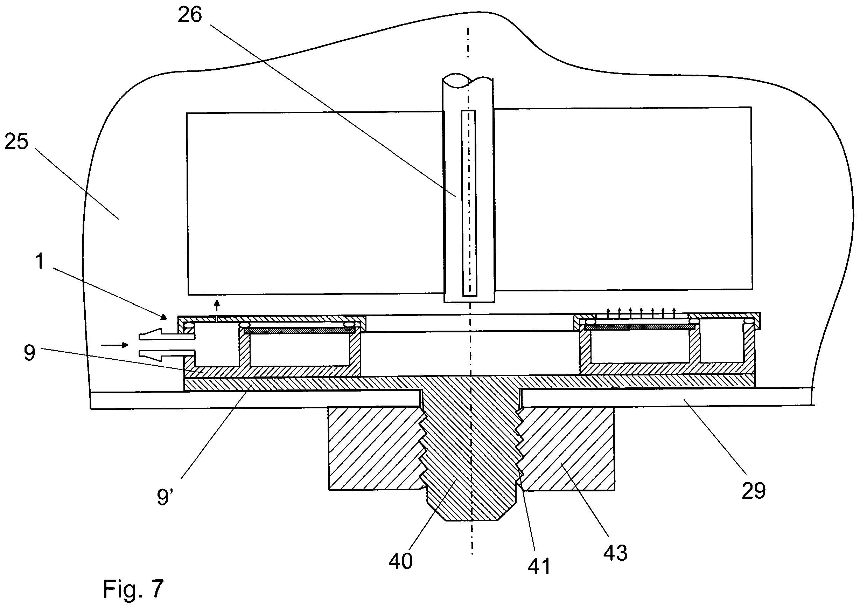

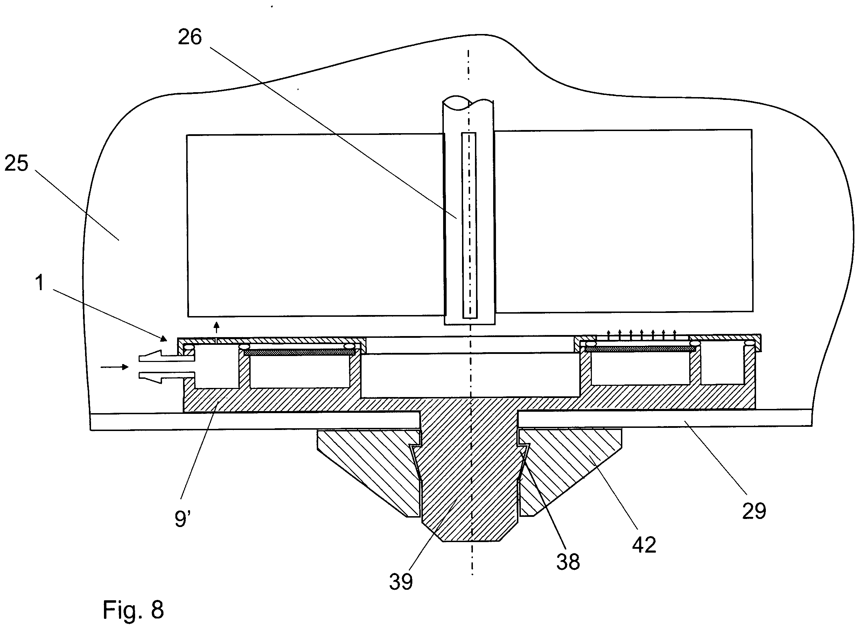

Bei weiteren Ausführungsformen nach den Figuren 7 und 8 kann die Begasungseinrichtung 1 über eine Schraub-, Bajonett-, Rast-, Klemm-, Kleb- oder Klippverbindung am Unterteil 5 der Begasungseinrichtung 1 ggf. unter Verwendung von Dichtelementen in einer kreisförmigen Öffnung im unteren Teil des Bioreaktors 25 fixiert werden und ggf. nach Benutzung ausgetauscht werden. Bei dieser Ausführungsform weist das Unterteil 5 einen durchgängigen, kreisförmigen Boden (9') auf. Der Boden 9' kann mit dem Boden 9 fest verbunden sein oder statt des in den Figuren 2 und 3 gezeigten ringförmigen Bodens 9 den Boden des Unterteils 5 bilden. Für das Einsetzen der Begasungsrichtung in den unteren Teil des Bioreaktors ist es vorteilhaft, wenn wenigstens ein Teil des Bodens 29 des Bioreaktors 25 um die kreisförmige Öffnung aus einem starren Material gefertigt ist. Durch Verrastung über die umlaufende Rastnase 38 oder Verschraubung über das Gewinde 41 des Zentralstücks 39, 40 der Begasungseinrichtung 1 mit dem Klemmteil 41, 42 wird die Begasungseinrichtung 1 fluiddicht in dem Bioreaktorboden 29 fixiert. Diese Ausführungsform erlaubt den flexiblen Einsatz und Austausch der Begasungseinrichtung sowohl in Einweg- als auch in Mehrwegbioreaktoren.

## Patentansprüche

1. Begasungsvorrichtung (1) für Bioreaktoren (25) mit einem ersten Begasungselement (3) mit Gasaustrittsöffnungen (36) einer ersten Größe und mit mindestens einem zweiten Begasungselement (4) mit Gasaustrittsöffnungen einer zweiten Größe, und wobei die Begasungselemente (3, 4) mit getrennten Begasungskanälen (7, 8) gebildet sind,
**dadurch gekennzeichnet,**
**dass** die Begasungselemente (3, 4) von einem gemeinsamen Gehäuse (2) gebildet werden,
**dass** das Gehäuse (2) als eine ringförmige Scheibe ausgebildet ist, in der die Begasungselemente (3, 4) konzentrisch zueinander angeordnet sind,
**dass** das Gehäuse (2) ein Unterteil (5) aufweist, in dem die Begasungskanäle (7,8) mit jeweils einem radial verlaufenden Zufluss (21, 23) angeordnet sind,
**dass** die Begasungskanäle (7,8) zueinander hin von einer Zwischenwandung (11) und in vertikaler Richtung nach unten von einem Boden (9) begrenzt sind.

2. Begasungsvorrichtung nach Anspruch 1,
**dadurch gekennzeichnet,**
**dass** das erste Begasungselement (3) als ein Ringsparger (16) mit Gasaustrittsöffnungen (36) von größer als 0,1 mm Durchmesser und das zweite Begasungselement (4) als ein Mikrosparger (15) mit Gasaustrittöffnungen (36) von kleiner als 0,1 mm Durchmesser ausgebildet ist.

3. Begasungsvorrichtung nach Anspruch 1,
**dadurch gekennzeichnet, dass** das erste Begasungselement (3) und das zweite Begasungselement (4) als Ringsparger (16) mit jeweils zwei verschieden großen Gasaustrittsöffnungen (36) ausgebildet ist, wobei die Gasaustrittsöffnungen (36) größer als 0,1 mm sind.

4. Begasungsvorrichtung nach Anspruch 1,
**dadurch gekennzeichnet, dass** das erste Begasungselement (3) und das zweite Begasungselement (4) als Microsparger (15) mit jeweils zwei verschieden großen Gasaustrittsöffnungen (36) ausgebildet ist, wobei die Gasaustrittsöffnungen (36) kleiner als 0,1 mm sind.

5. Begasungsvorrichtung nach Anspruch 2 oder 4,
**dadurch gekennzeichnet,**
**dass** die Gasaustrittsöffnungen des Mikrospargers (15) von einem porösen Material gebildet sind.

6. Begasungsvorrichtung nach einem der Ansprüche 1 bis 5,
**dadurch gekennzeichnet,**
**dass** das Unterteil (5) von einem Oberteil (6) abgedeckt ist, das den Begasungskanal (8) des Ringspargers (16) abdeckt und die Gasaustrittsöffnungen (36) aufweist.

7. Begasungsvorrichtung nach Anspruch 6,
**dadurch gekennzeichnet,**
**dass** das Oberteil (6) im Bereich des Mikrospargers (15) zum Begasungskanal (8) hin segmentförmige Öffnungen (17) aufweist, und
**dass** der Begasungskanal (8) von einem Belüftungsring (14) aus dem die Gasaustrittsöffnungen bildenden porösen Material abgedeckt ist.

8. Begasungsvorrichtung nach Anspruch 6 oder 7,
**dadurch gekennzeichnet,**
**dass** das Oberteil (6) gegenüber dem Unterteil (5) durch Dichtungen (18, 19, 20) abgedichtet ist.

9. Begasungsvorrichtung nach Anspruch 6 oder 7,
**dadurch gekennzeichnet,**
**dass** das Oberteil (6) mit dem Unterteil (5) verschweißt ist.

10. Begasungsvorrichtung nach Anspruch 6 oder 7,
**dadurch gekennzeichnet,**
**dass** das Oberteil (6) mit dem Unterteil (5) verklebt ist.

11. Begasungsvorrichtung nach einem der Ansprüche 1 bis 10,
**dadurch gekennzeichnet,**
**dass** die Zuflüsse (21, 23) zur unabhängigen Versorgung der Begasungskanäle (7, 8) jeweils einen Schlauchanschluss (22, 24) mit einem Rückschlagventil aufweisen.

12. Begasungsvorrichtung nach 1 bis 11,
**dadurch gekennzeichnet,**
**dass** das Gehäuse (2) eine zentrale, auf einen Rührerflansch (31) des Bioreaktors (25) abgestimmte Öffnung (32) aufweist und einem Rührer (26) vorgelagert am Boden (29) eines Innenraums (30) des Bioreaktors (25) anordenbar ist.

13. Begasungsvorrichtung nach einem der Ansprüche 1 bis 12,
**dadurch gekennzeichnet,**
**dass** das Unterteil (5) einen durchgehenden, kreisförmigen Boden (9') aufweist und durch Schraub-, Bajonett-, Rast-, Klemm-, Kleb- oder Klippverbindung dichtend in den Boden (29) des Bioreaktors (25) einsetzbar ist.

## Claims

1. A gassing device (1) for bioreactors (25) having a first gassing element (3) with gas discharge openings (36) of a first size and having at least one second gassing element (4) with gas discharge openings of a second size, and wherein the gassing elements (3, 4) are formed with separate gassing channels (7, 8),
**characterized in that** the gassing elements (3, 4) are formed by a common housing (2),
the housing (2) has the form of an annular disk in which
the gassing elements (3, 4) are arranged concentrically to each other,
the housing (2) has a lower part (5) in which are arranged
the gassing channels (7, 8) with in each case a radially extending inflow (21, 23)
the gassing channels (7, 8) are delimited relative to each other by a partition wall (11) and downwardly delimited in the vertical direction by a bottom (9).

2. The gassing device according to Claim 1,
**characterized in that**
the first gassing element (3) is a ring sparger (16) having gas discharge openings (36) of a size greater than 0.1 mm in diameter and the second gassing element (4) is a microsparger (15) having gas discharge openings (36) of a size less than 0.1 mm in diameter.

3. The gassing device according to Claim 1,
**characterized in that**
the first gassing element (3) and the second gassing element (4) are ring spargers (16) with in each case two differently sized gas discharge openings (36), the gas discharge openings (36) being larger than 0.1 mm.

4. The gassing device according to Claim 1,
**characterized in that** the first gassing element (3) and
the second gassing element (4) are microspargers (15) with in each case two differently sized gas discharge openings (36), the gas discharge openings (36) being smaller than 0.1 mm.

5. The gassing device according to Claim 2 or 4,
**characterized in that**
the gas discharge openings of the microsparger (15) are formed by a porous material.

6. The gassing device according to any of Claims 1 to 5,
**characterized in that**
the lower part (5) is covered by an upper part (6) that covers the gassing channel (8) of the ring sparger (16) and comprises the gas discharge openings (36).

7. The gassing device according to Claim 6,
**characterized in that**
in the region of the microsparger (15), the upper part (6) comprises segment-shaped openings (17) towards the gassing channel (8), and
the gassing channel (8) is covered by an gassing ring (14) made from the porous material forming the gas discharge openings.

8. The gassing device according to Claim 6 or 7,
**characterized in that**
the upper part (6) is sealed relative to the lower part (5) by seals (18, 19, 20).

9. The gassing device according to Claim 6 or 7,
**characterized in that**
the upper part (6) is welded to the lower part (5).

10. The gassing device according to Claim 6 or 7,
**characterized in that**
the upper part (6) is adhesively bonded to the lower part (5).

11. The gassing device according to any of Claims 1 to 10,
**characterized in that**
the inflows (21, 23) in each case comprise a tube connection (22, 24) with a non-return valve for independent supply of the gassing channels (7, 8).

12. The gassing device according to Claims 1 to 11,
**characterized in that**
the housing (2) has a central opening (32) matched to a stirrer flange (31) of the bioreactor (25) and may be arranged upstream of a stirrer (26) on a bottom (29) of an interior space (30) of the bioreactor (25).

13. The gassing device according to any of Claims 1 to 12,
**characterized in that**
the lower part (5) has a continuous, circular bottom (9') and may be inserted in a sealing manner into a bottom (29) of the bioreactor (25) by means of a screw, bayonet, latch, clamp, adhesive or clip connection.

## Revendications

1. Dispositif (1) d'apport de gaz pour des bioréacteurs (25), comprenant un premier élément (3) d'apport de gaz doté d'ouvertures (36) de sortie de gaz d'une première taille et au moins un deuxième élément (4) d'apport de gaz doté d'ouvertures de sortie de gaz d'une deuxième taille, et sachant que les éléments (3, 4) d'apport de gaz sont formés avec des canaux (7, 8) d'apport de gaz séparés,
**caractérisé en ce que** les éléments (3, 4) d'apport de gaz sont formés par un boîtier commun (2),
**en ce que** le boîtier (2) est réalisé sous la forme d'un disque annulaire dans lequel les éléments (3, 4) d'apport de gaz sont disposés concentriquement les uns par rapport aux autres,
**en ce que** le boîtier (2) présente une partie inférieure (5) dans laquelle les canaux (7, 8) d'apport de gaz sont disposés avec une arrivée respective (21, 23) s'étendant radialement,
**en ce que** les canaux (7, 8) d'apport de gaz sont délimités entre eux par une paroi intermédiaire (11) et en direction verticale vers le bas par un fond (9).

2. Dispositif d'apport de gaz selon la revendication 1,
**caractérisé en ce que** le premier élément (3) d'apport de gaz est réalisé sous la forme d'un aérateur annulaire (16) doté d'ouvertures (36) de sortie de gaz de diamètre supérieur à 0,1 mm, et le deuxième élément (4) d'apport de gaz est réalisé sous la forme d'un micro-aérateur (15) doté d'ouvertures (36) de sortie de gaz de diamètre inférieur à 0,1 mm.

3. Dispositif d'apport de gaz selon la revendication 1,
**caractérisé en ce que** le premier élément (3) d'apport de gaz et le deuxième élément (4) d'apport de gaz sont réalisés sous la forme d'aérateurs annulaires (16) dotés chacun de deux ouvertures (36) de sortie de gaz de tailles différentes, sachant que les ouvertures (36) de sortie de gaz sont supérieures à 0,1 mm.

4. Dispositif d'apport de gaz selon la revendication 1,
**caractérisé en ce que** le premier élément (3) d'apport de gaz et le deuxième élément (4) d'apport de gaz sont réalisés sous la forme de micro-aérateurs (15) dotés chacun de deux ouvertures (36) de sortie de gaz de tailles différentes, sachant que les ouvertures (36) de sortie de gaz sont inférieures à 0,1 mm.

5. Dispositif d'apport de gaz selon la revendication 2 ou 4, **caractérisé en ce que** les ouvertures de sortie de gaz du micro-aérateur (15) sont formées par un matériau poreux.

6. Dispositif d'apport de gaz selon l'une des revendications 1 à 5, **caractérisé en ce que** la partie inférieure (5) est recouverte par une partie supérieure (6) qui recouvre le canal (8) d'apport de gaz de l'aérateur annulaire (16) et qui présente les ouvertures (36) de sortie de gaz.

7. Dispositif d'apport de gaz selon la revendication 6,
**caractérisé en ce que** la partie supérieure (6) présente, dans la région du micro-aérateur (15), des ouvertures en forme de segments (17) en direction du canal (8) d'apport de gaz,
et **en ce que** le canal (8) d'apport de gaz est recouvert par une bague d'aération (14) constituée du matériau poreux formant les ouvertures de sortie de gaz.

8. Dispositif d'apport de gaz selon la revendication 6 ou 7, **caractérisé en ce que** la partie supérieure (6) est étanchée vis-à-vis de la partie inférieure (5) par des joints d'étanchéité (18, 19, 20).

9. Dispositif d'apport de gaz selon la revendication 6 ou 7, **caractérisé en ce que** la partie supérieure (6) est assemblée par soudage à la partie inférieure (5).

10. Dispositif d'apport de gaz selon la revendication 6 ou 7, **caractérisé en ce que** la partie supérieure (6) est assemblée par collage à la partie inférieure (5).

11. Dispositif d'apport de gaz selon l'une des revendications 1 à 10, **caractérisé en ce que** les arrivées (21, 23) présentent chacune, pour l'alimentation indépendante des canaux (7, 8) d'apport de gaz, un branchement (22, 24) de tuyau doté d'un clapet anti-retour.

12. Dispositif d'apport de gaz selon l'une des revendications 1 à 11, **caractérisé en ce que** le boîtier (2) présente une ouverture centrale (32) adaptée à un flasque d'agitateur (31) du bioréacteur (25), et il peut être disposé en amont d'un agitateur (26) sur le fond (29) d'un espace intérieur (30) du bioréacteur (25).

13. Dispositif d'apport de gaz selon l'une des revendications 1 à 12, **caractérisé en ce que** la partie inférieure (5) présente un fond circulaire continu (9'), et elle peut être insérée en étanchéité dans le fond (29) du bioréacteur (25) par un assemblage vissé, à baïonnette, par enclenchement, par serrage, par collage ou par clip.
